# FASCICULE DE BREVET EUROPEEN

(11) **EP 2 368 528 B1**
(45) Date de publication et mention de la délivrance du brevet: **08.07.2015**
(21) Numéro de dépôt: 11305335.9
(22) Date de dépôt: 25.03.2011
(51) Int. Cl.: A61F 2/44

(54) **Cage lombaire destinée à être insérée entre deux corps vertébraux et de forme générale anatomique pour remplir l'espace intervertébral**
Lendenwirbelgerüst zum Einsetzen zwischen zwei Wirbelkörper mit allgemeiner anatomischer Form zum Ausfüllen des Zwischenwirbelraums.
Lumbar cage intended for being inserted between two vertebral bodies with a general anatomic shape for filling the intervertebral space.

(30) Priorité: 25.03.2010 FR 1052188
(43) Date de publication de la demande: 28.09.2011
(73) Titulaire: Spineway, 69500 Bron (FR)
(72) Inventeur: Leroux, Stéphane, 69002, LYON (FR); Laurito, Philippe, 83143 Le Val (FR); Norotte, Gilles, 05000, GAP (FR)
(74) Mandataire: Thivillier, Patrick

(56) Documents cités:
- WO-A1-03/053290
- WO-A2-2005/055869
- WO-A2-2007/098288
- WO-A2-2008/102174
- FR-A1- 2 727 003
- GB-A- 2 454 229
- US-A1- 2003 153 975
- US-A1- 2005 060 035

## Description

L'invention se rattache au secteur technique des implants rachidiens.

Plus particulièrement, l'invention concerne une cage lombaire destinée à être insérée entre deux corps vertébraux, afin, notamment, de stabiliser les vertèbres lombaires et la charnière lombo-sacrée.

L'état de la technique, dans ce domaine, peut être illustré par l'enseignement du brevet FR 2.727.003 qui concerne un dispositif de stabilisation antérieure du rachis lombo-sacré.

Selon l'enseignement de ce brevet, ce dispositif comprend un élément ou cage apte à maintenir l'écartement entre les corps vertébraux, tout en étant agencé pour recevoir des greffons spongieux et permettre leur fusion intersomatique. La face antérieure de la cage présente deux trous orientés dont l'un est orienté vers le haut et l'autre vers le bas pour l'engagement de vis de fixation coopérant avec les corps vertébraux sus et sous-jacents. Il en résulte que l'insertion et la fixation de la cage s'effectue exclusivement par voie antérieure.

Une autre solution de ce type ressort de l'enseignement du document WO 2007/098288.

Le document WO 2008/102174 concerne une cage lombaire constituée de deux éléments montés avec capacité d'accouplement et dont l'un présente des agencements pour une insertion et fixation par la voie antérolatérale uniquement.

L'invention s'est fixée pour but de remédier à ces inconvénients d'une manière simple, sûre, efficace et rationnelle.

Le problème que se propose de résoudre l'invention est de pouvoir insérer la cage par voie latérale ou par voie antérieure selon l'étage considéré et l'anatomie.

Pour résoudre un tel problème, il a été conçu et mis au point une cage lombaire destinée à être insérée entre deux corps vertébraux et de forme générale anatomique pour remplir l'espace intervertébral.

Cette cage présente, sur une partie de sa périphérie :
- deux agencements aptes à coopérer séparément avec un appareil préhenseur pour permettre son insertion, par une voie antérieure ou par une voie antérolatérale ou par une voie latérale ;
- trois trous pour l'engagement de vis de fixation à savoir :
   - un trou central positionné entre les deux agencements, ledit trou étant orienté pour l'engagement d'une vis dirigée dans la vertèbre adjacente ;
   - un trou situé de l'un des côtés du trou central à proximité de l'un des agencements et du côté de la face latérale de ladite cage, ledit trou étant orienté pour l'engagement d'une vis dirigée dans la vertèbre adjacente, lorsque la cage est insérée par la voie antérieure ;
   - un trou situé de l'autre côté du trou central à proximité de l'autre agencement et du côté de la face antérieure de ladite cage, ledit trou étant orienté pour l'engagement d'une vis dirigée dans la vertèbre adjacente, lorsque la cage est insérée par la voie antérolatérale ;
   ladite cage étant fixée par au moins l'une de ces vis.

Afin d'assurer la préhension et l'impaction de la cage dans les trois directions, les agencements sont positionnés angulairement au niveau de chaque extrémité de l'une des faces de ladite cage. Les agencements sont constitués par des encoches en combinaison avec au moins un trou taraudé que présente ladite face.

Compte tenu du problème posé à résoudre, le trou central est positionné entre les deux encoches, chacun des deux autres trous étant positionné à proximité de l'une desdites encoches. L'un des trous pour l'engagement de la vis dirigée vers le bas ou vers le haut, est situé du côté de la face latérale, tandis que l'autre trou et situé du côté de la face antérieure.

Pour résoudre le problème posé d'éviter tout desserrage intempestif des vis, les têtes de vis et les trous présentent des agencements complémentaires de retenue. Les agencements sont constitués par un collet que présente, en débordement, la tête de vis et par un épaulement formé à l'intérieur du trou.

Selon d'autres caractéristiques, la cage présente :
- sur ses faces supérieure et inférieure, des stries ;
- deux ouvertures débouchantes et séparées aptes à recevoir des greffons osseux ;
- différents angles de lordose.

L'invention est exposée ci-après plus en détail à l'aide des figures des dessins annexés dans lesquels :
- la figure 1 est une vue en perspective de la cage lombaire selon l'invention ;
- la figure 2 est une vue en perspective de la cage montrant la mise en place des vis dans le cas d'une fixation antérolatérale ;
- la figure 3 est une vue de face correspondant à la figure 2 ;
- la figure 4 est une vue de dessous correspondant à la figure 2 ;
- la figure 5 est une vue en perspective de la cage montrant le positionnement des vis dans le cas d'une fixation antérieure ;
- la figure 6 est une vue de face correspondant à la figure 5 ;
- la figure 7 est une vue de dessous correspondant à la figure 5 ;
- la figure 8 est une vue de côté correspondant aux figures 4 ou 7.

La cage désignée dans son ensemble par (1) est de forme générale anatomique en étant dimensionnée pour bien remplir l'espace intervertébral avec pour objectif de maintenir l'écartement entre deux corps vertébraux de la partie lombaire du rachis. La cage (1) a une forme générale rectangulaire et présente différents angles de lordose.

D'une manière connue, les faces supérieure et/ou inférieure de la cage (1) peuvent présenter des stries (1a), afin d'éviter tout risque de recul. De même, la cage (1) peut présenter deux ouvertures débouchantes (1c) et (1d) séparées par une partie médiane pleine (le). D'une manière connue, ces ouvertures peuvent recevoir des greffons osseux.

Selon une caractéristique de l'invention, la cage lombaire (1) présente, sur une partie de sa périphérie, des agencements (1f) et (1g) aptes à coopérer séparément avec un appareil préhenseur pour permettre son insertion par une voie antérieure ou par une voie antérolatérale, et trois trous (1h), (1i) et (1j) pour l'engagement de vis de fixation (2), (3), (4) coopérant avec les corps vertébraux sus et sous-jacents, en fonction de l'une desdites voies choisies.

Comme il sera indiqué dans la suite de la description, la cage peut être fixée dans l'un des corps vertébraux par au moins l'une des vis (2), (3) ou (4).

Selon l'invention, la même cage (1) est conformée pour être posée selon plusieurs voies d'abord (antérieure, latérale, antérolatérale, ou autre). Dans ce but, la cage présente des agencements pour son insertion (préhension et impaction) selon ces différentes voies d'abord précitées.

Dans l'exemple illustré, la cage lombaire (1) présente deux agencements (1f) et (1g) positionnés angulairement au niveau de chaque extrémité de l'une des faces de ladite cage (1). Ces agencements sont constitués par des encoches en combinaison avec au moins un trou taraudé (1k) que présente la face à partir de laquelle sont formés lesdits agencements. Ces encoches (1f) et (1g) permettent, par conséquent, la préhension et l'impaction de la cage dans les deux directions (antérieure et antérolatérale). L'appareil préhenseur, de tout type connu et approprié, est vissé, par exemple, dans le trou taraudé (1k), tandis que des parties de l'appareil sont aptes à être positionnées dans les encoches latérales (1f) ou (1g), afin de parfaitement guider la cage, tout en la protégeant.

Comme indiqué, la cage présente trois trous, à savoir un trou central (1h) et deux trous latéraux (1i) et (1j) disposés de part et d'autre dudit trou central (1h).

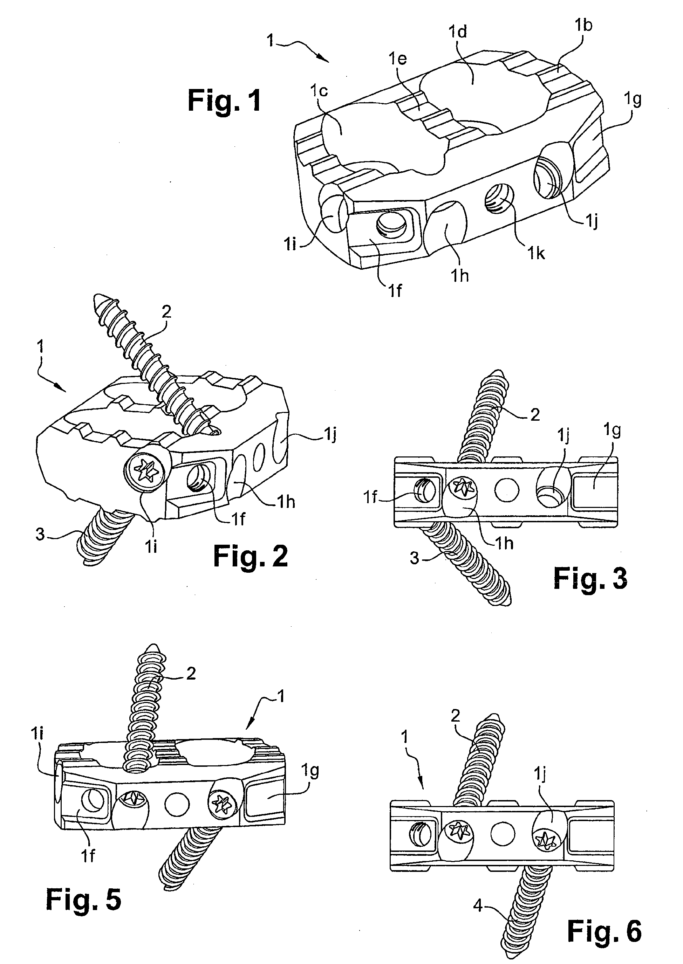

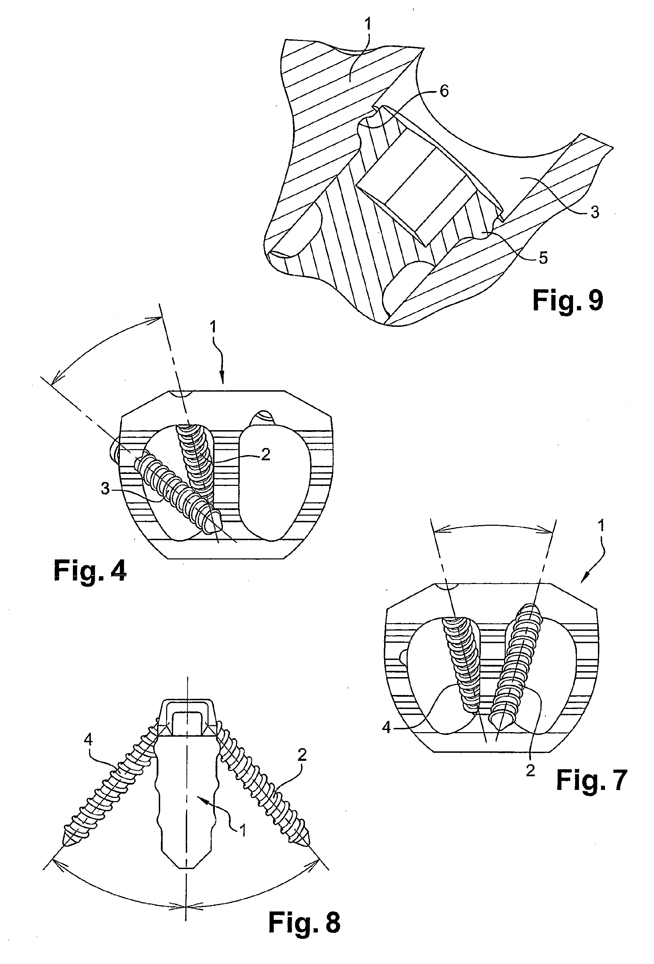

Dans une forme de réalisation et comme illustré dans les dessins, le trou central (1h) est orienté pour l'engagement d'une vis (2) dirigée vers le haut dans la vertèbre adjacente. Le trou (1i), situé de l'un des côtés du trou central (1h), est orienté afin de permettre l'engagement d'une vis (3) dirigée vers le bas de la vertèbre adjacente lorsque la cage est insérée par la voie antérolatérale. On renvoie aux figures 2, 3 et 4 qui montrent cette fixation antérolatérale.

Le trou (1j) est situé de l'autre côté du trou central (1h) et est orienté pour l'engagement d'une vis (4) dirigée vers le bas dans la vertèbre adjacente lorsque la cage (1) est insérée par la voie antérieure. On renvoie aux figures 5, 6 et 7.

Le trou central (1h) est positionné entre les deux encoches (1f) et (1g), tandis que chacun des deux autres trous (1i) et (1j) sont positionnés à proximité de l'une desdites encoches. Le trou (1i), pour l'engagement de la vis (3) est situé du côté de la face latérale de la cage (1), tandis que l'autre trou (1j) pour l'engagement de la vis (4) est situé du côté de la face antérieure.

Il apparaît donc que le trou central (1h) est commun aux deux directions pour une fixation antérolatérale ou une fixation antérieure.

Sans pour cela sortir du cadre de l'invention, d'autres formes de réalisation peuvent être envisagées au niveau de l'orientation, notamment des différents trous (1h), (1i) et (1j).

Par exemple :
- soit, le trou central (1h) est dirigé vers le bas, de même que les deux autres trous latéraux (1i) et (1j) ;
- soit le trou central (1h) est dirigé vers le haut, de même que les deux autres trous latéraux (1i) et (1j) ;
- soit le trou central (1h) est dirigé vers le haut, les deux trous latéraux (1i) et (1j) étant dirigés vers le bas ;
- soit le trou central (1h) est dirigé vers le bas, les deux autres trous latéraux (1i) et (1j) étant dirigé vers le haut.

Compte tenu de ces dispositions, il en résulte plusieurs solutions pour la direction des vis.

Par exemple, à titre indicatif nullement limitatif :
- Des vis dirigées vers le bas au niveau de (L5-S1), une vis étant dirigée dans le sacrum permettant un meilleur ancrage et une facilité de mise en place, étant donné que l'accès est meilleur que pour une insertion de vis vers le haut.
- Des vis toutes orientées dans la même vertèbre que ce soit celle du haut ou celle du bas, afin de créer un ensemble dynamique. Cette solution autorise des micromouvements au sein de l'unité spinale et fonctionnelle. La vertèbre non fixée pourra être légèrement mobile diminuant ainsi les risques de cisaillement sur la ou les vis. Cet ensemble dynamique permet de diminuer la durée de la prise de greffe et d'améliorer la repousse osseuse.

Selon une autre caractéristique, les têtes de vis (2a), (3a) et (4a) présentent des agencements complémentaires de retenue pour éviter tout desserrage intempestif. Par exemple, ces agencements sont constitués par un collet (5) qui présente, en débordement, la tête de vis correspondante et par un épaulement (6) formé à l'intérieur du trou correspondant.

Avantageusement, la cage (1) est réalisée dans un matériau plastique notamment en PEEK. Les vis peuvent être en alliage de titane.

Bien évidemment, les coques, selon les caractéristiques de l'invention, peuvent être réalisées dans différentes tailles, au niveau de la longueur et de la largeur, et selon différentes hauteurs en fonction notamment des étages considérés.

Les avantages ressortent bien de la description, en particulier on souligne et on rappelle la possibilité d'insérer et de fixer la cage lombaire par la voie antérieure, ou antérolatérale, ou latérale.

Les différentes possibilités de fixation par des voies d'abord, sont adaptées à l'étage considéré et en fonction de l'anatomie permettant de conserver le ligament longitudinal antérieur, compte tenu de l'état du patient.

Selon l'état du patient et en fonction de l'étage considéré et de l'anatomie, le ligament longitudinal antérieur peut être conservé grâce aux différentes possibilités de fixation offertes par la cage.

## Revendications

1. Cage lombaire (1) destinée à être insérée entre deux corps vertébraux et de forme générale anatomique pour remplir l'espace intervertébral, **caractérisée en ce qu'**elle présente, sur une partie de sa périphérie :
- deux agencements (1f) et (1g) aptes à coopérer séparément avec un appareil préhenseur pour permettre son insertion, par une voie antérieure ou par une voie antérolatérale ou par une voie latérale ;
- trois trous (1h), (1i), (1j) pour l'engagement de vis de fixation à savoir :
• un trou central (1h) positionné entre les deux agencements (1f) et (1g), ledit trou étant orienté pour l'engagement d'une vis (2) dirigée dans la vertèbre adjacente ;
• un trou (1i) situé de l'un des côtés du trou central (1h) à proximité de l'un des agencements (1f) et du côté de la face latérale de ladite cage, ledit trou étant orienté pour l'engagement d'une vis dirigée (3) dans la vertèbre adjacente, lorsque la cage (1) est insérée par la voie antérieure ;
• un trou (1j) situé de l'autre côté du trou central (1h) à proximité de l'autre agencement (1g) et du côté de la face antérieure de ladite cage, ledit trou étant orienté pour l'engagement d'une vis (4) dirigée dans la vertèbre adjacente, lorsque la cage est insérée par la voie antérolatérale ;
ladite cage étant fixée par au moins l'une de ces vis (2) et/ou (3) et/ou (4) dans au moins une vertèbre.

2. Cage selon la revendication 1, **caractérisée en ce que** le trou central (1h) est orienté vers le haut, tandis que les deux trous latéraux (1i) et (1j) sont orientés vers le bas.

3. Cage selon la revendication 1, **caractérisée en ce que** le trou central (1h) est orienté vers le bas, tandis que les deux trous latéraux (1i) et (1j) sont orientés vers le haut.

4. Cage selon la revendication 1, **caractérisée en ce que** le trou central (1h) et les deux trous latéraux (1i) et (1j) sont orientés vers le bas.

5. Cage selon la revendication 1, **caractérisée en ce que** le trou central (1h) et les deux trous latéraux (1i) et (1j) sont orientés vers le haut.

6. Cage selon l'une des revendications 1 à 5, **caractérisée en ce que** les agencements (1f) et (1g) sont positionnés angulairement au niveau de chaque extrémité de l'une des faces de ladite cage.

7. Cage selon la revendication 6, **caractérisée en ce que** les agencements (1f) et (1g) sont constitués par des encoches en combinaison avec au moins un trou taraudé (1k) que présente ladite face.

8. Cage selon l'une quelconque des revendications 1 à 7, **caractérisée en ce que** les têtes de vis et les trous (1h), (1i) et (1j) présentent des agencements complémentaires de retenue afin d'éviter tout desserrage intempestif.

9. Cage selon la revendication 8, **caractérisée en ce que** les agencements sont constitués par un collet (5) que présente, en débordement, la tête de vis et par un épaulement (6) formé à l'intérieur du trou.

10. Cage selon l'une quelconque des revendications 1 à 9, **caractérisée en ce qu'**elle présente, sur ses faces supérieure et inférieure, des stries (1b).

11. Cage selon l'une quelconque des revendications 1 à 10, **caractérisée en ce qu'**elle présente deux ouvertures débouchantes et séparées (1c) et (1d) aptes à recevoir des greffons osseux.

12. Cage selon l'une quelconque des revendications 1 à 11, **caractérisée en ce qu'**elle présente différents angles de lordose.

## Patentansprüche

1. Lendenwirbelkäfig (1), der dazu bestimmt ist, zwischen zwei Wirbelkörpern eingefügt zu werden und eine allgemeine anatomische Form aufweist, um den Zwischenwirbelraum auszufüllen, **dadurch gekennzeichnet, dass** er auf einem Teil seiner Peripherie aufweist:
- zwei Anordnungen (1f) und (1g), die geeignet sind, getrennt mit einem Greifapparat zusammenzuwirken, um ein Einfügen durch einen vorderen Weg oder durch einen vorderen seitlichen Weg oder durch einen seitlichen Weg zu erlauben;
- drei Löcher (1h), (1i), (1j) für das Eingreifen von Befestigungsschrauben, und zwar:
• ein zentrales Loch (1h), das zwischen den zwei Anordnungen (1f) und (1g) positioniert ist, wobei das genannte Loch für das Eingreifen einer Schraube (2) ausgerichtet ist, die in den danebenliegenden Wirbelkörper gerichtet ist;
• ein Loch (1i), das sich auf einer der Seiten des zentralen Lochs (1h) in der Nähe einer der Anordnungen (1f) und auf der Seite der lateralen Fläche des genannten Käfigs befindet, wobei das genannte Loch für das Eingreifen einer Schraube (3) ausgerichtet ist, die in den danebenliegenden Wirbel gerichtet ist, wenn der Käfig (1) durch den vorderen Weg eingefügt wird;
• ein Loch (1j), das sich auf der anderen Seite des zentralen Lochs (1h) in der Nähe der anderen Anordnung (1g) und der Seite der vorderen Fläche des genannten Käfigs befindet, wobei das genannte Loch für das Eingreifen einer Schraube (4) ausgerichtet ist, die in den danebenliegenden Wirbel gerichtet ist, wenn der Käfig durch den vorderen seitlichen Weg eingefügt wird;
wobei der genannte Käfig durch wenigstens eine dieser Schrauben (2) und / oder (3) und / oder (4) in wenigstens einem Wirbel befestigt ist.

2. Käfig gemäß Anspruch 1, **dadurch gekennzeichnet, dass** das zentrale Loch (1h) nach oben ausgerichtet ist, während die zwei lateralen Löcher (1i) und (1j) nach unten ausgerichtet sind.

3. Käfig gemäß Anspruch 1, **dadurch gekennzeichnet, dass** das zentrale Loch (1h) nach unten ausgerichtet ist, während die zwei lateralen Löcher (1i) und (1j) nach oben ausgerichtet sind.

4. Käfig gemäß Anspruch 1, **dadurch gekennzeichnet, dass** das zentrale Loch (1h) und die zwei lateralen Löcher (1i) und (1j) nach unten ausgerichtet sind.

5. Käfig gemäß Anspruch 1, **dadurch gekennzeichnet, dass** das zentrale Loch (1h) und die zwei lateralen Löcher (1i) und (1j) nach oben ausgerichtet sind.

6. Käfig gemäß einem der Ansprüche 1 bis 5, **dadurch gekennzeichnet, dass** die Anordnungen (1f) und (1g) an jedem Ende einer der Flächen des genannten Käfigs winkelförmig positioniert sind.

7. Käfig gemäß Anspruch 6, **dadurch gekennzeichnet, dass** die Anordnungen (1f) und (1g) durch Auskerbungen in Verbindung mit wenigstens einer Gewindebohrung (1k) gebildet sind, die die genannte Seite aufweist.

8. Käfig gemäß einem der Ansprüche 1 bis 7, **dadurch gekennzeichnet, dass** die Schraubenköpfe und die Löcher (1h), (1i) und (1j) komplementäre Rückhalteanordnungen aufweisen, um jegliches versehentliches Lösen zu verhindern.

9. Käfig gemäß Anspruch 8, **dadurch gekennzeichnet, dass** die Anordnungen durch eine Struppe (5), die der Schraubenkopf am Überstand aufweist, und durch einen Absatz (6), der im Innern des Lochs gebildet ist, gebildet sind.

10. Käfig gemäß einem der Ansprüche 1 bis 9, **dadurch gekennzeichnet, dass** er an seinen oberen und unteren Seiten Riefelungen (1b) aufweist.

11. Käfig gemäß einem der Ansprüche 1 bis 10, **dadurch gekennzeichnet, dass** er zwei einmündende und getrennte Öffnungen (1c) und (1d) aufweist, die geeignet sind, Knochenimplantate aufzunehmen.

12. Käfig gemäß einem der Ansprüche 1 bis 11, **dadurch gekennzeichnet, dass** er unterschiedliche Lordosewinkel aufweist.

## Claims

1. Lumbar cage (1) designed to be inserted between two vertebral bodies with a general anatomic shape to fill the intervertebral space, **characterised in that** it comprises, on a part of its periphery:
- Two arrangements (If) and (1g) that can separately easily work with a gripper device to enable its insertion through an anterior path or an anterolateral path or a lateral path;
- Three holes (1h), (1i), (1j) for the engagement of fixing screws, i.e.:
• A central hole (1h) located between the two arrangements (If) and (1g). The said hole is oriented for the engagement of a screw (2) directed into the adjacent vertebra;
• A hole (1i) situated on one of the sides of the central hole (1h) near one of the arrangements (1f) and on the side of the lateral face of the said cage; the said hole is oriented for engagement of a screw directed (3) into the adjacent vertebra, when the cage (1) is inserted through the anterior path;
• A hole (1j) situated on the other side of the central hole (1h) near the other arrangement (1g) and on the side of the anterior face of the said cage; the said hole is oriented for engagement of a screw (4) directed into the adjacent vertebra, when the cage is inserted through the anterolateral path;
The said cage is fixed by at least one of these screws (2) and/or (3) and/or (4) in at least one vertebra.

2. Cage according to claim 1, **characterised in that** the central hole (1h) is oriented upwards, while the two lateral holes (1i) and (1j) are oriented downwards.

3. Cage according to claim 1, **characterised in that** the central hole (1h) is oriented downwards, while the two lateral holes (1i) and (1j) are oriented upwards.

4. Cage according to claim 1, **characterised in that** the central hole (1h) and the two lateral holes (1i) and (1j) are oriented downwards.

5. Cage according to claim 1, **characterised in that** the central hole (1h) and the two lateral holes (1i) and (1j) are oriented upwards.

6. Cage according to one of the claims 1 to 5, **characterised in that** the arrangements (1f) and (1g) are angularly positioned at each end of one of the sides of the said cage.

7. Cage according to claim 6, **characterised in that** the arrangements (1f) and (1g) are formed by notches in combination with at least one threaded hole (1k) that is seen on the said face.

8. Cage according to any one of the claims 1 to 7, **characterised in that** the screw heads and the holes (1h), (1i) and (1j) have complementary holding arrangements to prevent any unintended loosening.

9. Cage according to claim 8, **characterised in that** the arrangements consist of a collar (5) that shows, in overflow, the screw head and a shoulder (6) formed inside the hole.

10. Cage according to any one of the claims 1 to 9, **characterised in that** it has grooves (1b) on its upper and lower faces.

11. Cage according to any one of the claims 1 to 10, **characterised in that** it has two through and separate openings (1c) and (1d) for receiving bone grafts.

12. Cage according to any one of the claims 1 to 11, **characterised in that** it has different angles of lordosis.
